# FASCICULE DE BREVET EUROPEEN

(11) **EP 1 027 092 B1**
(45) Date de publication et mention de la délivrance du brevet: **01.09.2004**
(21) Numéro de dépôt: 98952817.9
(22) Date de dépôt: 29.10.1998
(51) Int. Cl.: A61M 35/00, B65D 47/42

(54) **APPLICATEUR CUTANE DE LIQUIDE**
GERÄT ZUM AUFTRAGEN VON FLÜSSIGKEITEN AUF DIE HAUT
LIQUID APPLICATOR FOR THE SKIN

(30) Priorité: 30.10.1997 FR 9713654
(43) Date de publication de la demande: 16.08.2000
(73) Titulaire: EMERIT, Michel, 95110 Sannois (FR); PATERNOTTE, Yanick, 95110 Sannois (FR)
(72) Inventeur: EMERIT, Michel, 95110 Sannois (FR); PATERNOTTE, Yanick, 95110 Sannois (FR)
(74) Mandataire: Blot, Philippe Robert Emile
(86) Numéro de dépôt international: PCT/FR1998/002321
(87) Numéro de publication internationale: WO 1999/022801

(56) Documents cités:
- WO-A-91/12197
- WO-A-91/13814
- WO-A-94/13352
- FR-A- 1 415 759
- GB-A- 2 020 174
- US-A- 3 148 401
- US-A- 3 876 314

## Description

La présente invention concerne un applicateur cutané de liquide, du type comportant un tampon hydrophile d'application du liquide sur une zone à traiter et des moyens de liaison du tampon à une cartouche de stockage du liquide, lesquels moyens de liaison sont adaptés pour un montage du tampon en regard d'une sortie d'écoulement du liquide hors de la cartouche.

Il est courant d'utiliser un tampon hydrophile pour appliquer sur une blessure un liquide désinfectant ou une substance médicamenteuse.

Afin d'éviter un dessèchement trop rapide du tampon hydrophile, durant son stockage, il est connu de stocker séparément le tampon hydrophile et le liquide destiné à imprégner le tampon.

Le document FR-A-2.732.585 décrit par exemple un emballage comportant deux compartiments initialement séparés dans lesquels sont disposés d'une part un tampon hydrophile et d'autre part un produit actif liquide. Avant l'utilisation du tampon, les deux compartiments sont mis en communication, de sorte que le produit actif liquide imbibe le tampon.

Dans ce type d'emballage, afin de conserver les propriétés actives du liquide contenu dans l'un des compartiments, il est nécessaire de garantir une asepsie de l'ensemble de l'emballage. De plus, il est nécessaire, afin de pouvoir proposer un tel conditionnement à la vente, d'obtenir des certifications garantissant l'asepsie et la fiabilité de l'emballage. De telles certifications doivent être obtenues pour chaque emballage correspondant à un type différent de conditionnement, ces derniers pouvant varier notamment par leur forme, ou leur volume. Ainsi, l'obtention de ces certificats est une opération coûteuse et longue qui augmente considérablement le prix unitaire du produit.

Les documents WO-91/12197, WO-94/13352, US-3 876 314, US-3,148,401, FR-A-1.415.759 et GB-A-2.020.174 décrivent des applicateurs de liquide du type précité. Toutefois, dans ces applicateurs, le tampon hydrophile d'application du liquide est formé par un bloc de mousse hydrophile de faible surface rendant mal aisée l'application du liquide sur une grande surface.

L'invention a pour but de proposer un applicateur cutané, adapté pour recevoir un produit actif liquide conditionné séparément, ne nécessitant pas l'obtention de certificats spécifiques pour l'applicateur associé au conteneur de stockage du liquide indépendamment certifié, et permettant une application commode du liquide sur une blessure.

A cet effet, l'invention a pour objet un applicateur cutané de liquide du type précité, caractérisé en ce que le tampon comporte une bande hydrophile repliée sur elle-même et les moyens de liaison comportent des moyens de maintien de la sortie d'écoulement de la cartouche enchâssés entre les deux bords rabattus de la bande.

Suivant des modes particuliers de réalisation, l'applicateur comporte l'une ou plusieurs des caractéristiques suivantes :
- les moyens de liaison comportent un collier de fixation sur le goulot d'une cartouche ;
- le collier de fixation comporte deux brides identiques accouplées l'une à l'autre et délimitant chacune un demi collier ;
- les deux brides sont liées par soudage, rivetage, sertissage, ou mise en oeuvre d'au moins une patte de liaison, venue de matière avec une bride et repliée sur l'autre bride ;
- les moyens de liaison comportent des moyens d'écartement des bords de la bande hydrophile dans la zone de liaison sur la cartouche ;
- chaque bride est prolongée, de part et d'autre de la sortie d'écoulement de la cartouche, par des griffes de retenue des extrémités de la bande hydrophile ;
- le tampon comporte un support flexible sur lequel est fixée une bande hydrophile, et le support flexible est solidaire des moyens de liaison ;
- le support flexible a généralement la forme d'un arceau, délimitant un logement de réception de la sortie d'écoulement de la cartouche, et éventuellement d'un bouchon d'obturation de celle-ci ;
- le support flexible est d'épaisseur progressivement décroissante depuis les moyens de liaison jusqu'à son extrémité éloignée des moyens de liaison ;
- les moyens de liaison sont adaptés pour permettre un mouvement relatif entre l'applicateur et la cartouche afin de permettre la rupture d'un bouchon d'obturation de la sortie d'écoulement de la cartouche ;
- il comporte un organe de perforation d'une face d'extrémité perforable de la cartouche ;
- les moyens de liaison comportent un filetage adapté pour coopérer avec un filetage complémentaire de la cartouche, afin d'assurer ledit mouvement relatif entre l'applicateur et la cartouche ;
- ledit mouvement relatif entre l'applicateur et la cartouche est un mouvement de rotation ; et
- l'applicateur est conditionné dans un sachet protecteur.

L'invention a également pour objet un nécessaire de soins, caractérisé en ce qu'il comporte au moins une cartouche hermétiquement scellée contenant un liquide de traitement aseptique et au moins un applicateur tel que défini ci-dessus, un applicateur étant adapté pour être monté sur une cartouche, laquelle cartouche est initialement séparée de l'applicateur.

L'invention a en outre pour objet un dispositif de soins, caractérisé en ce qu'il comporte un applicateur tel que défini ci-dessus et une cartouche scellée contenant un liquide de traitement aseptique sur laquelle est monté l'applicateur.

L'invention sera mieux comprise à la lecture de la description qui va suivre, donnée uniquement à titre d'exemple et faite en se référant aux dessins, sur lesquels :
- les figures 1 et 2 sont des vues en élévation respectivement de face et de côté d'un dispositif de soins comportant un applicateur selon l'invention monté sur une cartouche ;
- la figure 3 est une vue en élévation d'une cartouche à usage unique destinée à recevoir un applicateur selon l'invention ;
- la figure 4 est une vue en coupe d'un flan portant une bande hydrophile utilisé pour former l'applicateur, la coupe étant effectuée dans un plan transversal médian ;
- la figure 5 est une vue en élévation d'un flan de l'applicateur avant montage de la bande hydrophile ;
- la figure 6 est une vue de dessous de la structure de support de l'applicateur représentée sans la bande hydrophile.
- la figure 7 est une vue en élévation de face d'une variante de réalisation du dispositif de soins comportant un applicateur selon l'invention ;
- la figure 8 est une vue en perspective avec arrachement d'une autre variante de réalisation du dispositif de soins ; et
- la figure 9 est une vue en perspective d'encore une variante de réalisation du dispositif de soins.

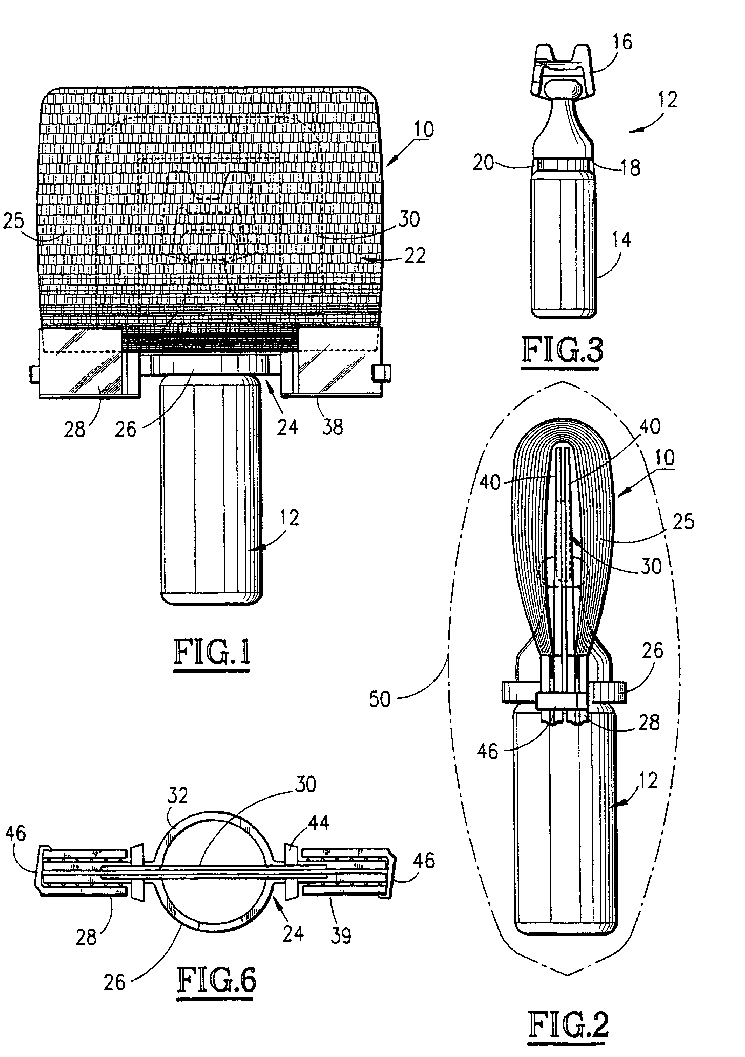

Sur les figures 1 et 2, est représenté un applicateur cutané 10 monté sur une cartouche 12 de stockage d'un produit actif liquide aseptique tel qu'un désinfectant ou une substance médicamenteuse. La cartouche est représentée seule sur la figure 3.

De telles cartouches sont bien connues et sont actuellement largement diffusées. Elles comportent par exemple un corps flexible sensiblement cylindrique 14 réalisé en matière plastique moulée. Le corps est prolongé à sa partie supérieure par un bouchon 16 venu de matière avec le corps.

Au voisinage du bouchon 16, le corps 14 comporte généralement une gorge périphérique 18 au travers de laquelle s'étend éventuellement un voile de matière plastique 20 produit lors de l'assemblage des deux demi-coquilles formant la cartouche. Entre le bouchon 16 et la gorge 18, la cartouche comporte un tronçon progressivement rétréci formant un goulot.

La contenance de tels cartouches est adaptée pour renfermer la quantité nécessaire de liquide pour un usage unique. Cette contenance est comprise entre 1 ml et 5 ml et est par exemple égale à 2,5 ml, sans que ces valeurs soient considérées comme limitatives.

L'applicateur 10 représenté sur la figure 1 comporte essentiellement un tampon hydrophile 22 et des moyens 24 de liaison du tampon 22 sur la cartouche de stockage 12 en regard d'une sortie d'écoulement du liquide hors de celle-ci. Le tampon hydrophile 22 comporte une bande hydrophile 25, par exemple de la gaze.

Les moyens de liaison 24 comportent deux flans conformés identiques et accolés. Ils sont réalisés en polymère, par exemple en polypropylène ou en polyéthylène. Ils forment, une fois assemblés, un collier 26 de montage sur la cartouche, des moyens 28 de maintien de la bande hydrophile 25 et un arceau 30 de support de cette bande hydrophile.

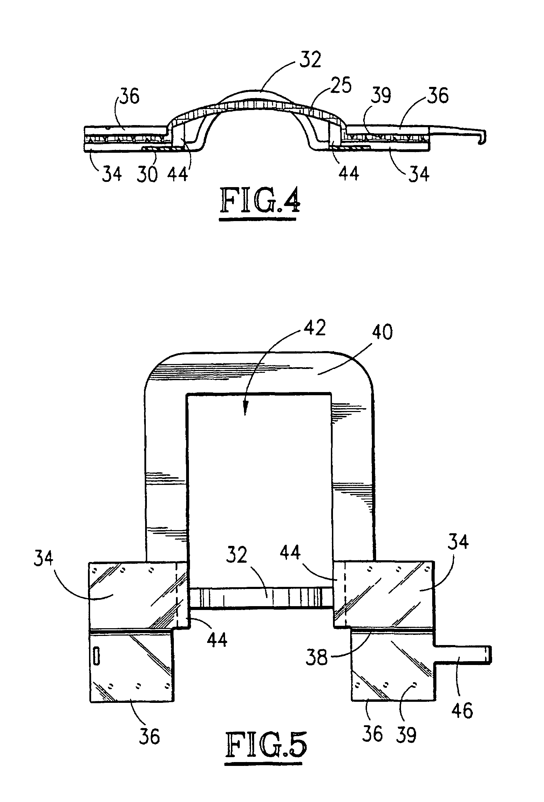

L'un des flans est représenté avant assemblage des deux flans sur les figures 4 et 5.

Chaque flan comporte un demi-collier 32 sensiblement en forme de demi-cercle et dont la hauteur est légèrement inférieure à la largeur de la gorge 18. Le demi-collier est prolongé à chaque extrémité par deux pans coplanaires 34 de forme rectangulaire. Chaque pan 34 est associé, dans sa partie inférieure, à un rabat 36 venu de matière et relié au pan 34 par une ligne de pliage 38. Les surfaces en regard des pans 34 et des rabats 36 sont munies de picots 39 répartis suivant des motifs conjugués. Ils sont adaptés pour le maintien de la bande hydrophile 25. Ainsi, les pans 34 sur lesquels sont repliés les rabats 36 forment, comme représenté sur la figure 4, des griffes de retenue de la bande 25.

Dans leur partie supérieure, les deux pans 34 sont reliés l'un à l'autre par une arche 40 venue de matière, qui a une forme générale de U. Cette arche délimite, avec le demi-collier 32, un espace évidé rectangulaire 42. Ce dernier a une taille suffisamment grande pour recevoir le goulot de la cartouche ainsi que son bouchon 16.

Comme représenté sur la figure 2, l'épaisseur de l'arche 40 décroît progressivement depuis les pans 34 jusqu'au pontet transversal d'extrémité reliant les deux jambes de l'arche.

En outre, de part et d'autre du demi-collier 32, les pans 34 comportent, de préférence, des ressauts latéraux rapportés 44 faisant saillie par rapport à la surface des pans 34 et de l'arche 40 du même côté que le demi-collier 32.

Enfin, l'un des rabats 36 comporte sur une paroi latérale extérieure une patte de liaison 46 recourbée dont la longueur correspond sensiblement à trois fois l'épaisseur des pans 34.

A partir des deux flans identiques représentés aux figures 4 et 5 et d'une bande hydrophile 25, l'applicateur est réalisé de la manière suivante. Les deux flans sont appliqués l'un sur l'autre autour du goulot d'une cartouche 12 scellée, de sorte que les arches 40 et les pans 34 se superposent deux à deux, les deux demi-colliers 32 délimitant le collier 26 reçu dans la gorge 18 de la cartouche. La bande hydrophile 25 est alors repliée de part et d'autre de l'arceau 30 formé par les arches 40 accouplés. La longueur de la bande 25 est choisie de sorte que les extrémités repliées de celle-ci viennent au contact des picots 39 portés par les pans 34, tout en restant à distance des lignes de pliage 38. Ainsi, la bande hydrophile 25 définit de part et d'autre de l'arceau 30 des bords rabattus qui forment deux plages opposées d'application du produit liquide.

La bande hydrophile étant ainsi positionnée, les quatre rabats 36 sont repliés sur les extrémités de la bande hydrophile 25 autour des lignes de pliage 38.

Les pattes 46 sont ensuite rabattues de part et d'autre des deux flancs accolés, de sorte que l'extrémité recourbée des pattes 46 vient en appui sur la face arrière des rabats du flan opposé. Ainsi, les rabats assurent la retenue de la bande hydrophile, alors que les pattes 46 assurent la solidarisation des deux flans et le maintien de l'applicateur sur la cartouche.

L'applicateur 10 est retenu axialement sur la cartouche par le collier 26 engagé dans la gorge 18. Toutefois, le collier autorise la rotation de l'applicateur autour de l'axe de la cartouche.

Afin de permettre la réception du voile 20, les deux demi-colliers 32 se relient l'un à l'autre par une zone de pincement de largeur progressivement décroissante, apte à recevoir le voile 20.

Le montage de l'applicateur sur la cartouche est réalisé dans des conditions d'asepsie requises, de même que leur conditionnement dans un emballage tel qu'un sachet 50 en matière plastique transparente, représenté sur la figure 2. Les conditions d'asepsie imposées pour le sachet 50 sont moins contraignantes que celles imposées pour la cartouche 12, puisque ce sachet n'est pas destiné à contenir directement une substance active.

On comprend que, le corps de la cartouche étant maintenu entre les doigts, avec interposition du sachet ou autre emballage, un mouvement de rotation de l'applicateur 10 autour de l'axe de la cartouche 12, sous l'action des doigts sollicitant les pans 34, provoque le cisaillement du bouchon et ainsi l'ouverture de la cartouche. Le liquide contenu dans celle-ci est alors libre de s'écouler pour imbiber la bande hydrophile, notamment sous l'action d'une pression manuelle exercée sur le corps flexible de la cartouche.

Le tampon étant ainsi imprégné, l'utilisateur peut le dégager de son emballage et badigeonner la zone à traiter avec le tampon, tout en ne maintenant que le corps de la cartouche. Le produit liquide est appliqué par mise en contact de la zone à traiter avec l'un ou l'autre des bords rabattus opposés de la bande qui forment les plages exposées d'application du liquide.

L'applicateur cutané peut aussi être vendu séparément de la cartouche à laquelle il doit être associé en vue de son utilisation.

Il est alors conditionné seul dans un emballage approprié. L'applicateur est fabriqué comme précédemment, à l'exception de la première étape dans laquelle les deux flans sont accolés sans interposition d'une cartouche.

L'applicateur ainsi formé peut alors être mis en place ultérieurement sur une cartouche comme représenté sur les figures 1 et 2. Les ressauts 44 assurent, comme représenté sur la figure 4, un écartement des extrémités de la bande 25 dans la zone du collier 26. Les extrémités de la bande ainsi espacées l'une de l'autre délimitent un passage d'introduction du bouchon 16 d'une cartouche. La cartouche est introduite dans l'applicateur par déformation élastique du goulot de la cartouche et du collier 26.

Le bouchon d'extrémité 16 obturant la sortie d'écoulement du fluide est reçu après montage dans l'espace rectangulaire 42 délimité par l'arceau 30, comme représenté sur les figures 1 et 2.

La sortie d'écoulement de la cartouche est alors enchâssée entre les deux bords rabattus de la bande 25.

L'applicateur peut également être commercialisé sous la forme d'un nécessaire de soins comportant un ou plusieurs applicateurs associés à un nombre correspondant de cartouches scellées, les cartouches étant initialement séparées des applicateurs.

Les cartouches sur lesquelles l'applicateur selon l'invention est destiné à être appliqué sont largement commercialisées et ont fait l'objet d'obtention de certificats garantissant leur fiabilité et leur asepsie. Aussi, l'utilisation de ces cartouches courantes associées à un applicateur permet de fournir un dispositif de soins satisfaisant aux conditions d'hygiène nécessaires à un usage satisfaisant. De plus, ce dispositif de soins ne nécessite pas de requérir de nouvelles certifications en vue de sa distribution.

L'arceau 30 étant d'épaisseur progressivement décroissante vers son extrémité éloignée de la cartouche, le tampon est plus souple à son extrémité destinée à entrer en contact avec la peau qu'à son extrémité de fixation sur la cartouche, ce qui facilite l'application.

De plus, le confinement des extrémités de la bande hydrophile entre les pans 34 et les rabats 36 reliés entre eux par les lignes de pliage 38 assure une canalisation du liquide en excès dans la bande hydrophile, évitant ainsi que le liquide ne goutte sur la cartouche ou les doigts de l'utilisateur.

Dans l'exemple décrit, les flans ont une structure permettant un très bon maintien de la bande hydrophile. Cependant, ceux-ci peuvent être de structure plus simple et par exemple ne pas comporter de rabat. La bande hydrophile est alors maintenue enserrée directement entre les pans 34, ou fixée directement sur ceux-ci.

De même, les flans peuvent être reliés l'un à l'autre par soudage, rivetage, sertissage, ou tout autre moyen approprié en remplacement de la mise en oeuvre de la patte 46. Les deux flans peuvent également être venus de matière, notamment dans la zone supérieure de l'arceau.

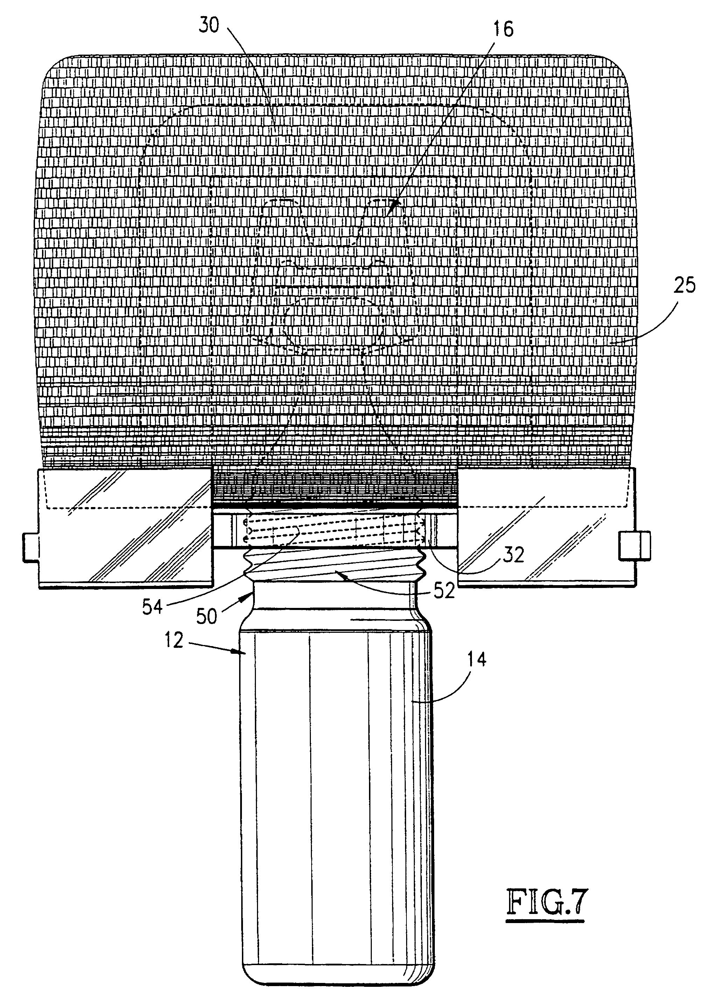

Dans les variantes de réalisation décrites aux figures 7 à 9, les parties identiques ou analogues à celles du mode de réalisation précédent sont désignées par les mêmes numéros de référence.

Dans la variante de réalisation de la figure 7, la cartouche de stockage 12 comporte, dans la région reliant le corps 14 au goulot, un tronçon cylindrique 50 pourvu extérieurement d'un filetage 52 formé d'un filet hélicoïdal venu de matière avec la cartouche.

Chaque demi-collier 32 comporte intérieurement sur son tronçon en forme de demi-cercle une nervure hélicoïdale 54 formant un filetage adapté pour coopérer avec le filetage complémentaire 52 porté par la cartouche 12.

Dans ce mode de réalisation, comme précédemment, la sortie d'écoulement du liquide hors de la cartouche est initialement obturée par un bouchon 16 venu de matière avec le corps, ce bouchon 16 pouvant être détaché par cisaillement.

Initialement, l'applicateur est engagé à l'extrémité libre du filetage 52. Le bouchon 16 est alors disposé dans l'arceau 30 entre les deux bords rabattus de la bande hydrophile 25.

Afin d'imbiber la bande hydrophile 25 en vue de l'application du liquide contenu dans la cartouche 12 sur une zone à traiter, l'utilisateur visse l'applicateur sur la cartouche 12. Ce vissage provoque le rapprochement axial de l'applicateur 10 et de la cartouche 12. Lors du mouvement hélicoïdal de l'applicateur par rapport à la cartouche 12 résultant du vissage, le bouchon 16 maintenu entre les doigts de l'opérateur est cisaillé, ce qui provoque l'ouverture de la cartouche. Ainsi, le liquide contenu dans celle-ci est libre de s'écouler jusqu'à la bande hydrophile 25.

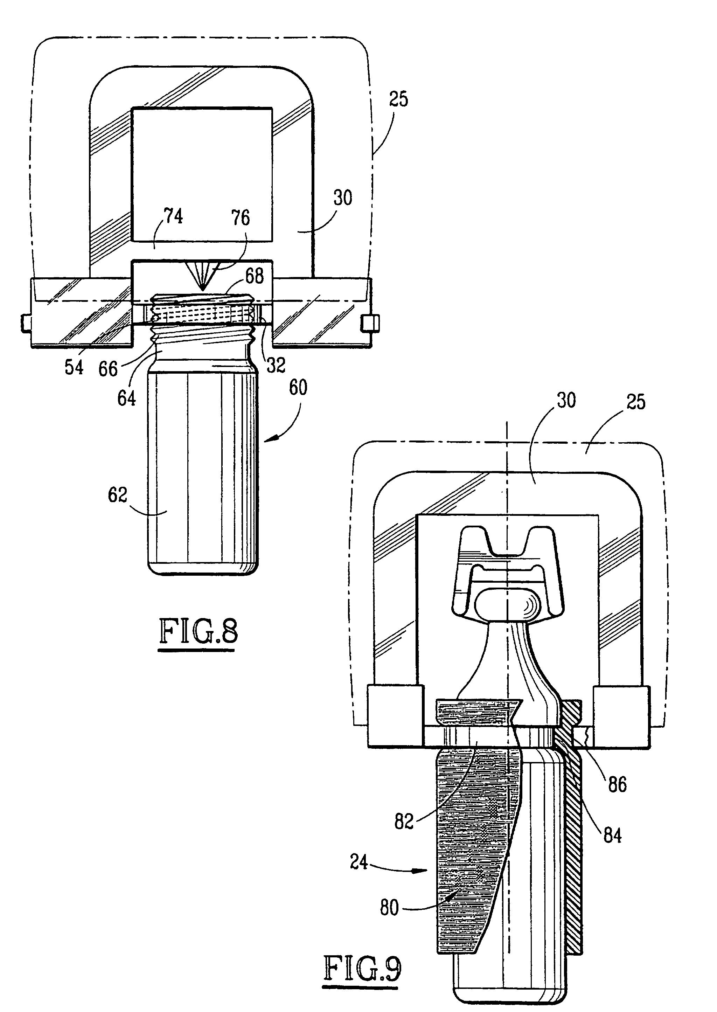

Dans le mode de réalisation de la figure 8, la cartouche, notée 60 comporte un corps 62 et un goulot 64 pourvus extérieurement d'un filetage 66. Le goulot 64 est obturé par une face d'extrémité perforable 68.

Dans ce mode de réalisation, les moyens 24 de liaison de la bande hydrophile 25 à la cartouche 62 sont analogues à ceux du mode de réalisation de la figure 7. Ainsi, une nervure hélicoïdale 52 est prévue intérieurement sur chaque demi-collier 32 pour coopérer avec le filetage 66.

L'arceau 30 porte une traverse 74 s'étendant diamétralement par rapport à la cartouche 60. La traverse 74 est munie, en son milieu, d'un organe de perforation 76 orienté vers la face perforable 68 de la cartouche. L'organe de perforation 76 s'étend suivant l'axe du filetage 66.

L'organe de perforation 76 est constitué d'une pointe nervurée, c'est-à-dire d'un élément de forme généralement conique, suivant des génératrices duquel, s'étendent des nervures convergeant au sommet du cône.

Avec un tel agencement, lors du vissage de l'applicateur sur le goulot 66, l'organe de perforation 76 sollicite la face perforable 68 et provoque sa perforation par enfoncement progressif de l'organe 76 à surface cannelée. Une fois l'organe 76 au moins partiellement enfoncé dans la face 68, le liquide contenu dans la cartouche 62 est libre de s'écouler dans les canaux définis entre les nervures convergentes de l'organe 76. Le liquide s'écoule alors entre les deux bords rabattus de la bande hydrophile 25 et imprégne celle-ci permettant d'appliquer le liquide sur la zone à traiter.

L'applicateur cutané représenté sur la figure 9 est adapté pour être monté sur une cartouche 12 du type de celle représentée sur la figure 3.

Dans cette variante de réalisation, les moyens de liaison 24 comportent un manchon élastique 80, autour de l'axe duquel la bande hydrophile 25 est montée rotative par l'intermédiaire d'une bague 82 portant l'arceau de support 30.

Le manchon 80 est réalisé dans une matière élastiquement déformable. Il présente intérieurement un diamètre légèrement inférieur au diamètre externe du corps 14 de la cartouche. Il comporte sur sa paroi intérieure un bourrelet périphérique 84 destiné à être reçu dans la gorge 20 de la cartouche.

A son extrémité supérieure, la face externe du manchon 80 comporte une gorge 86 dans laquelle la bague 82 est montée rotative autour de l'axe commun du manchon 80 et de la cartouche 14. La bague 82, formée dans un matériau rigide. Elle porte l'arceau 30 suivant un plan diamétral.

Avec un tel agencement, le manchon élastique 80 est emmanché à force autour du corps de la cartouche 14 jusqu'à ce que le bombage périphérique 84 soit reçu dans la gorge périphérique 80. Dans cette position, le bouchon 16 de la cartouche est entouré par l'arceau 30 et est disposé entre les deux bords rabattus de la bande hydrophile 25. Ainsi, pour provoquer l'ouverture de la cartouche 12, l'opérateur déplace angulairement la bande hydrophile 25 et le bouchon 16 par rapport au corps 14 de la cartouche. Le bouchon 16 se trouve cisaillé, libérant ainsi le liquide contenu dans la cartouche.

Dans tous les modes de réalisation décrits ici, les plages d'application du liquide sur la zone à traiter sont formées par les faces exposées des bords rabattus de la bande hydrophile 25. Ainsi, ces plages ont une grande étendue puisqu'elles sont formées par presque toute la surface exposée de la bande. L'application de liquide sur une zone à traiter de grande étendue est donc facile à réaliser, l'essentiel de la surface de la bande hydrophile pouvant être utilisée pour appliquer le liquide actif.

## Revendications

1. Applicateur cutané de liquide, comportant un tampon hydrophile (22) d'application de liquide sur une zone à traiter et des moyens (24) de liaison du tampon (22) à une cartouche (12) de stockage du liquide, lesquels moyens de liaison (24) sont adaptés pour un montage du tampon (22) en regard d'une sortie d'écoulement du liquide hors de la cartouche (12), dans laquelle le tampon (22) comporte une bande hydrophile (25) repliée sur elle-même et les moyens de liaison (24) comportent des moyens (28) de maintien de la sortie d'écoulement de la cartouche enchâssés entre les deux bords rabattus de la bande (25), **caractérisé en ce que** les moyens de liaison (24) sont adaptés pour permettre un mouvement relatif entre l'applicateur (10) et la cartouche (12) afin de permettre la rupture d'un bouchon (16) d'obturation de la sortie d'écoulement de la cartouche (12).

2. Applicateur selon la revendication 1, **caractérisé en ce que** les moyens de liaison (24) comportent un collier (26) de fixation sur le goulot d'une cartouche.

3. Applicateur selon la revendication 2, **caractérisé en ce que** le collier (26) de fixation comporte deux brides identiques (32) accouplées l'une à l'autre et délimitant chacune un demi collier.

4. Applicateur selon la revendication 3, **caractérisé en ce que** les deux brides sont liées par soudage, rivetage, sertissage, ou mise en oeuvre d'au moins une patte de liaison (46), venue de matière avec une bride et repliée sur l'autre bride.

5. Applicateur selon l'une quelconque des revendications précédentes, **caractérisé en ce que** les moyens de liaison (24) comportent des moyens (44) d'écartement des bords de la bande hydrophile (25) dans la zone de liaison sur la cartouche.

6. Applicateur selon la revendication 2, **caractérisé en ce que** chaque bride (32) est prolongée, de part et d'autre de la sortie d'écoulement de la cartouche, par des griffes (28) de retenue des extrémités de la bande hydrophile (25).

7. Applicateur selon l'une quelconque des revendications précédentes, **caractérisé en ce que** le tampon (22) comporte un support flexible (30) sur lequel est fixée une bande hydrophile (25), et **en ce que** le support flexible (30) est solidaire des moyens de liaison (24).

8. Applicateur selon la revendication 7, **caractérisé en ce que** le support flexible (30) a généralement la forme d'un arceau, délimitant un logement (42) de réception de la sortie d'écoulement de la cartouche, et éventuellement d'un bouchon (16) d'obturation de celle-ci.

9. Applicateur selon les revendications 7 ou 8, **caractérisé en ce que** le support flexible (30) est d'épaisseur progressivement décroissante depuis les moyens de liaison (24) jusqu'à son extrémité éloignée des moyens de liaison (24).

10. Applicateur selon l'une quelconque des revendications précédentes, **caractérisé en ce qu'**il comporte un organe (76) de perforation d'une face d'extrémité perforable (68) de la cartouche (60).

11. Applicateur selon l'une quelconque des revendications précédentes, **caractérisé** en ce les moyens de liaison (24) comportent un filetage (54) adapté pour coopérer avec un filetage complémentaire (52, 66) de la cartouche (12, 60) afin d'assurer ledit mouvement relatif entre l'applicateur et la cartouche.

12. Applicateur selon l'une quelconque des revendications 1 à 10, **caractérisé en ce que** ledit mouvement relatif entre l'applicateur (10) et la cartouche (12) est un mouvement de rotation.

13. Applicateur selon l'une quelconque des revendications précédentes, **caractérisé en ce que** l'applicateur est conditionné dans un sachet protecteur (50).

14. Applicateur selon l'une quelconque des revendications précédentes, **caractérisé en ce que** la bande hydrophile (25) définit deux plages opposées d'application du produit liquide.

15. Nécessaire de soins, **caractérisé en ce qu'**il comporte au moins une cartouche (12) hermétiquement scellée contenant un liquide de traitement aseptique et au moins un applicateur (10) selon l'une quelconque des revendications précédentes, un applicateur étant adapté pour être monté sur une cartouche, laquelle cartouche est initialement séparée de l'applicateur.

16. Dispositif de soins, **caractérisé en ce qu'**il comporte un applicateur (10) selon l'une quelconque des revendications 1 à 14, et une cartouche (12) hermétiquement scellée contenant un liquide aseptique et sur laquelle est monté ledit applicateur.

## Patentansprüche

1. Geräte zum Auftragen von Flüssigkeiten auf die Haut, mit einem hydrophilen Tampon (22) zur Aufbringung der Flüssigkeit auf eine zu behandelnde Zone und Mitteln (24) zur Verbindung des Tampon (22) mit einer Kartusche (12) zur Aufnahme der Flüssigkeit, welche Verbindungsmittel (24) geeignet sind zur Montage des Tampon (22) in Bezug auf die Auslaßöffnung der Flüssigkeit an der Kartusche (12), wobei der Tampon (22) ein hydrophiles Band (25) umfaßt, und die Verbindungsmittel (24) Mittel (28) aufweisen, die die Auslaßöffnung der Kartusche eingefügt zwischen den beiden umgeschlagenen Rändern des Bandes (25) halten, **dadurch gekennzeichnet, dass** die Verbindungsmittel (24) so ausgebildet sind, dass sie eine relative Bewegung zwischen dem Gerät (10) und der Kartusche (12) zur Brechung eines Verschlußstopfes der Auslaßöffnung der Kartusche (12) gestatten.

2. Gerät nach Anspruch 1, **dadurch gekennzeichnet, dass** die Verbindungsmittel einen Ring zur Befestigung am Hals einer Kartusche umfassen.

3. Gerät nach Anspruch 2, **dadurch gekennzeichnet. dass** der Ring (26) zwei identische Bügel (32) umfaßt, die miteinander verbunden sind und jeweils einen Halbring bilden.

4. Gerät nach Anspruch 3, **dadurch gekennzeichnet, dass** die beiden Bügel durch Schweißen, Nieten oder eine Verbindungsklammer verbunden sind, die einstückig mit einem Bügel hergestellt und über den anderen Bügel gelegt sind.

5. Gerät nach einem der vorhergehenden Ansprüche, **dadurch gekennzeichnet, dass** die Verbindungsmittel (24) Mittel (44) zum Trennen der Ränder des hydrophilen Bandes (25) in der Verbindungszone mit der Kartusche aufweisen.

6. Gerät nach Anspruch 2, **dadurch gekennzeichnet, dass** jeder Bügel beiderseits der Auslaßöffnung der Kartusche durch Klammern (28) zum Halten der Enden des hydrophilen Bandes (25) verlängert ist.

7. Gerät nach einem der vorhergehenden Ansprüche, **dadurch gekennzeichnet, dass** der Tampon (22) eine flexible Stütze (30) umfaßt, an der ein hydrophiles Band (25) befestigt ist, und dass die flexible Stütze (30) mit den Verbindungsmitteln (24) verbunden ist.

8. Gerät nach Anspruch 7, **dadurch gekennzeichnet, dass** die flexible Stütze (30) im wesentlichen die Form eines Bogens aufweist und einen Sitz (42) für die Aufnahme der Auslaßöffnung der Kartusche und gegebenenfalls eines Verschlußstopfes (16) der Kartusche bildet.

9. Gerät nach Anspruch 7 oder 8, **dadurch gekennzeichnet, dass** die flexible Stütze (30) eine progressiv abnehmende Stärke von den Verbindungsmitteln (24) zu dem von den Verbindungsmitteln (24) entfernten Ende aufweist.

10. Gerät nach einem der vorhergehenden Ansprüche, **dadurch gekennzeichnet, dass** es ein Perforationsorgan (76) für ein perforierbares Ende (68) der Kartusche (60) aufweist.

11. Gerät nach einem der vorhergehenden Ansprüche, **dadurch gekennzeichnet, dass** die Verbindungsmittel (24) eine Gewinde (54) umfassen, das vorgesehen ist zum Zusammenwirken mit einem komplementären Gewinde (52,66) der Kartusche (12,60) zur Sicherung der Relativbewegung zwischen dem Gerät und der Kartusche.

12. Gerät nach einem der Ansprüche 1 bis 10, **dadurch gekennzeichnet, dass** die Relativbewegung zwischen dem Gerät (10) und der Kartusche (12) eine Rotationsbewegung ist.

13. Gerät nach einem der vorhergehenden Ansprüche, **dadurch gekennzeichnet, dass** das Gerät in einem Schutzbeutel angeordnet ist.

14. Gerät nach einem der vorhergehenden Ansprüche, **dadurch gekennzeichnet, dass** das hydrophile Band zwei gegenüberliegende Flächen zur Aufbringung des flüssigen Produktes bildet.

15. Gebrauchssatz, **dadurch gekennzeichnet. dass** er wenigstens eine Kartusche (12) umfaßt, die hermetisch versiegelt ist und eine aseptische Behandlungsflüssigkeit enthält, und wenigstens ein Gerät (10) zur Aufbringung gemäß einem der vorhergehenden Ansprüche, wobei das Aufbringungsgerät zur Montage an der Kartusche vorgesehen ist, welche Kartusche anfangs von dem Aufbringungsgerät getrennt ist.

16. Behandlungsvorrichtung, **dadurch gekennzeichnet, dass** sie ein Aufbringungsgerät (10) gemäß einem der Ansprüche 1 bis 14 und einen hermetisch verschlossene Kartusche (12) umfaßt, die eine aseptische Flüssigkeit enthält und an der das Aufbringungsgerät montierbar ist.

## Claims

1. Applicator for applying liquid to the skin, including a hydrophilic pad (22) for applying liquid to an area to be treated and means (24) for connecting the pad (22) to a cartridge (12) for storing the liquid, which connecting means (24) are adapted so that the pad (22) can be mounted opposite an outlet through which the .Liquid flows out of the cartridge (12), in which the pad (22) comprises a hydrophilic strip (25) folded on itself and the connecting means (24) include means (28) for holding the outlet of the cartridge between the two folded edges of the strip (25), **characterized in that** the connecting means (24) allow relative movement between the applicator (10) and the cartridge (12) to enable a stopper (16) blocking the outlet of the cartridge (12) to be broken off.

2. Applicator according to Claim 1, **characterized in that** the connecting means (24) include a collar (26) adapted to be fixed to the neck of a cartridge.

3. Applicator according to Claim 2, **characterized in that** the fixing collar (26) includes two identical flanges (32) fastened together and each delimiting, a half-collar.

4. Applicator according to Claim 3, **characterized in that** the two flanges are welded, riveted or crimped together or fixed together by at least one connecting lug (46) in one piece with one flange and bent over the other flange.

5. Applicator according to any one of the preceding claims, **characterized in that** the connecting means (24) include means (44) for spreading the edges of the hydrophilic strip (25) in the area of connection to the cartridge.

6. Applicator according to Claim 2, **characterized in that** each flange (32) is extended on either side of the outlet of the cartridge by claws (28) for retaining the ends of the hydrophilic strip (25).

7. Applicator according to any one of the preceding claims, **characterized in that** the pad (22) includes a flexible support (30) to which a hydrophilic strip (25) is fixed and **in that** the flexible support (30) is attached to the connecting means (24).

8. Applicator according to Claim 7, **characterized in that** the flexible support (30) is in the general form of a hoop delimiting a housing (42) to receive the outlet of the cartridge and possibly a stopper (16) for closing off the latter.

9. Applicator according to Claim 7 or Claim 8, **characterized in that** the thickness of the flexible support (30) decreases progressively from the connecting means (24) to its end remote from the connecting means (24).

10. Applicator according to any one of the preceding claims, **characterized in that** it includes a member (76) for perforating an end face (68) of the cartridge (60) which is adapted to be perforated.

11. Applicator according to any one of the preceding claims, **characterized in that** the connecting means (24) include a screwthread (54) adapted to cooperate with a complementary screwthread (52, 66) of the cartridge (12, 60) to bring about the said relative movement between the applicator and the cartridge.

12. Applicator according to any one of Claims 1 to 10, **characterized in that** the said relative movement between the applicator (10) and the cartridge (12) is a rotational movement.

13. Applicator according to any one of the preceding claims, **characterized in that** the applicator is packaged in a protective sachet (50).

14. Applicator according to any one of the preceding claims, **characterized in that** the hydrophilic strip (25) defines two opposite areas for applying the liquid product.

15. Therapeutic kit **characterized in that** it includes at least one hermetically sealed cartridge (12) containing a sterile treatment liquid and at least one applicator (10) according to any any one of the preceding claims, adapted to be mounted on a cartridge which is initially separate from the applicator.

16. Therapeutic device **characterized in that** it includes an applicator (10) according to any one of Claims 1 to 14 and a hermetically sealed cartridge (12) containing a sterile liquid and on which the said applicator is mounted.
